Europäisches Patentamt

**European Patent Office**

**Office européen des brevets**

(11) Publication number: **0 243 548**
A1

## EUROPEAN PATENT APPLICATION

(21) Application number: 86303338.7

(22) Date of filing: 01.05.86

(51) Int. Cl.⁴ **A61K 39/012** , A61K 47/00

(43) Date of publication of application:
04.11.87 Bulletin 87/45

(84) Designated Contracting States:
**AT BE CH DE FR GB IT LI LU NL SE**

(71) Applicant: **Lee, Eng-Hong**
**121 Bagot Street, Unit No. 3**
**Guelph Ontario N1H 5T8(CA)**

(72) Inventor: **Lee, Eng-Hong**
**121 Bagot Street, Unit No. 3**
**Guelph Ontario N1H 5T8(CA)**

(74) Representative: **Warren, Anthony Robert et al**
**BARON & WARREN 18 South End Kensington**
**London W8 5BU(GB)**

(54) A vaccine for immunization against coccidiosis.

(57) A vaccine can be used for immunizing chickens and other domestic animals of the avian species from coccidiosis. Oocysts of one or more coccidia are diluted in a 1.5% solution of carrageenan. The oocysts are uniformly suspended and maintain a uniform level of infection throughout the solution. The chickens or other animals to be vaccinated can be exposed to the vaccine by placing the animals in the same room as the vaccine and allowing the animals to drink the vaccine as they desire.

EP 0 243 548 A1

## A VACCINE FOR IMMUNIZATION AGAINST COCCIDIOSIS

This invention relates to a vaccine for immunizing poultry against a disease known as coccidiosis. In particular, this invention relates to a vaccine for immunizing poultry against coccidiosis whereby poultry is placed in a room or area with the vaccine and consume the vaccine orally at their own initiative.

A method of immunizing chickens against cecal coccidiosis is disclosed in U.S. Patent #3,147,186 naming Edgar as inventor and being issued on September 1st, 1964. Unfortunately, the method described in the Edgar patent does not result in uniform infection of chickens when sporulated oocysts are added to feed water. Also, the method proposed by Edgar can require extensive and lengthy monitoring of the chickens and of fecal droppings. It is inconvenient and impractical, when vaccinating large flocks of poultry, to inoculate each animal separately by manually inserting a controlled dosage of oocysts orally.

It is an object of the present invention to provide a vaccine for immunizing poultry whereby the poultry can consume the vaccine orally at their own initiative.

In accordance with the present invention, a vaccine, which can be taken orally, for immunizing domestic animals of the avian species from coccidiosis is characterized by oocysts of at least one coccidium in relation to which immunization is desired. The oocysts of the coccidium are diluted in a solution which results in uniform suspension and infection of said oocysts. Preferably, the solution contains a nonsticky, edible gum. Still more preferably, the solution is approximately a 1.5% solution.

Eimeria tenella can be obtained in accordance with the procedure outlined in a prior publication by E. H. Lee, O. Remmler and M. A. Fernando, entitled "Sexual Differentiation in Eimeria tenella, (1977) 63 Journal of Parasitology, pages 155 and 156. Other species of coccidia that cause coccidiosis that can be used in accordance with this invention are Eimeria necatrix, Eimeria hagani, Eimeria acervulina, Eimeria maxima, Eimeria brunetti, Eimeria proecox , Eimeria mivati and Eimeria mitis. The method of obtaining each of these species is known by those skilled in the art and is not further described herein.

It has been discovered that oocysts of Eimeria tenella and of other coccidia remain uniformly suspended in a solution of a non-sticky edible gum. Some edible gums, for example, guar gum, are too sticky and trap or kill the sporulated oocysts of coccidia. This will result in a greatly reduced level of infectivity of the coccidia that are suspended in the sticky gum solution. There are therefore two requirements of the aqueous solution of non-sticky gum. Firstly, the oocysts of the coccidium must be uniformly suspended in the solution. Secondly, the level of infectivity of the oocysts in the solution must not be significantly reduced from the level of infectivity of the oocysts before they are added to the solution.

It has been discovered through experimentation that oocysts of Eimeria tenella and of other coccidia will remain uniformly suspended in a carrageenan solution and that the solution will not have any significant effect on the level of infectivity of the oocysts. Preferably, the carrageenan solution contains 1.5 grams of carrageenan per 100 millilitres of water.

Since the oocysts of a coccidium are uniformly distributed in the non-sticky gum solution, the oocysts can be administered to poultry by having the poultry ingest the solution orally at their own initiative. It is not necessary to administer the solution to each animal manually. The poultry can be left in any suitably enclosed area or room where they are able to drink the solution containing the oocysts. It has been found that four to twelve day old poultry offspring will drink essentially the same amount of the solution when the offspring have been placed in an enclosed area or room with equal access to the solution for a controlled time period. Therefore, as long as the oocysts are uniformly distributed and the solution does not interfere with the level of infectivity of said oocysts, the offspring will be uniformly inoculated by the vaccine. As the age of the offspring exceeds twelve days, a pecking order can be established and the offspring will no longer consume an essentially equal amount of solution even though they have equal access. In that event, some chicks will ingest too many oocysts and some may not ingest any at all, even though the oocysts are uniformly suspended in said solution.

While in the examples, only one coccidium is used in the solution at one time, it is possible to use two or more species of coccidia simultaneously. Eimeria tenella and Eimeria necatrix are used in the examples because they are the two most pathogenic species among the six commonly found coccidia which cause coccidiosis in chickens. They are, however, the two least immunogenic species, that is, they produce the least protection against immunization. Therefore, if an immunization method is effective against either Eimeria tenella or Eimeria necatrix, it will be effective for the remaining species as well. Eimeria tenella is the preferred species for experimentation, partly, not only, because it is more prevalent than Eimeria necatrix and is the main cause of death among chickens suffering from coccidiosis, but also, because Eimeria tenella appears almost exclusively in the ceca of chickens instead of infecting all over the intestines like other species. As the infection is confined to one particular area of the chicken, the damage or lesion can be accurately scored.

As can be seen from Table 1, sporulated oocysts of Eimeria tenella suspended in a 1.5% solution of carrageenan (i.e. 1.5 grams of carrageenan per 100 millilitres of water) could be stored up to 6 months

Table 1 - Uniform suspension of Eimeria tenella oocysts in 1.5% carrageenan solution.

Average of Means

| Time Lapse | Experiment #1 | Experiment #2 | Experiment #3 | Experiment #4 | Experiment #5 | Experiment #6 |
|---|---|---|---|---|---|---|
| 0 hour | 97 | 110 | 101 | 107 | 101 (10) | 106 |
| 1 hour | -- | -- | 96 | 115 | -- | -- |
| 2 hours | 91 | 91 | 98 | 88 | -- | -- |
| 3 hours | 119 | 105 | 97 | 89 | -- | -- |
| 4 hours | -- | -- | 94 | 97 | -- | -- |
| 20 hours | 91 | 94 | -- | -- | -- | -- |
| 24 hours | -- | -- | 113 | 105 | -- | -- |
| 20 days | -- | -- | -- | -- | 98 (6) | -- |
| 6 months | -- | -- | -- | -- | -- | 94 |
| means | $1.5 \times 10^4$ | $1.3 \times 10^4$ | $1.4 \times 10^4$ | $1.4 \times 10^4$ | $2.1 \times 10^4$ | $3.5 \times 10^5$ |

Each number represents 4 replicates except Experiment #5. Numbers in parenthesis in Experiment #5 represent numbers of samples counted in 2 replicates each.

while remaining within plus or minus 15% of the mean number of oocysts. It is therefore not necessary to use the vaccine as soon as the oocysts are added to the non-sticky edible gum solution. However, as will be seen later, it is preferred that the solution be in liquid form as opposed to gel form. If the solution is in gel form at any time after the oocysts are added, it cannot be reconverted to liquid form as the increase in

4

temperature necessary to reconvert the solution to liquid form will kill at least some of the oocysts thereby significantly reducing the level of infectivity of said oocysts in said solution.

As indicated in Table 2, it was found that the amount of a 1.5% solution of carrageenan, containing 1.5 grams of carrageenan per 100 millilitres of water, had no effect on the infectivity of Eimeria tenella in chickens. In other words, the level of infectivity of Eimeria tenella remained the same regardless of the volume of non-sticky edible gum solution used. Therefore, the carrageenan solution does not trap or kill the sporozoites of the coccidium and does not reduce the level of infectivity of the oocysts in said solution.

Preferably, the controlled time period ranges from thirty minutes to four hours and, still more preferably, from one to three hours. It has been found that the best results are obtained when the controlled time period or exposure time ranges from one to three hours with consecutive exposure times being at least eighteen hours apart from one another. It is often convenient to have consecutive exposure times twenty-four hours apart from one another.

It has also been found that better resistance to coccidiosis is achieved when there is more than one exposure time. Preferably, the number of exposure times ranges from two to four.

The use of the vaccine in accordance with the invention is illustrated in the following examples but the invention is not limited thereto. A suitable level of infectivity for vaccination purposes of Eimeria tenella is 300 oocysts per millilitre of solution containing 1.5 grams of non-sticky edible gum per 100 millilitres of water. A suitable level of infectivity for Eimeria necatrix for vaccination purposes is 200 oocysts per millilitre of a solution containing 1.5 grams of non-sticky edible gum per 100 millilitres of water.

Table 2 - Effect of the amount of carrageenan on the
uniformity in infectivity of Eimeria tenella
in chickens.

| Number of Oocysts | Millilitres of 1.5% Carrageenan | Mean Lesion Score ± S. D. |
|---|---|---|
| 1 X 10⁴ | 0.25 | 5.5 ± 1.5 |
| 1 X 10⁴ | 0.5 | 4.6 ± 1.3 |
| 1 X 10⁴ | 1.0 | 6.2 ± 1.4 |

Five birds per group.

EXAMPLE #1

IMMUNIZATION OF CHICKENS AGAINST EIMERIA TENELLA WITH OOCYSTS SUSPENDED IN A 1.5% CARRAGEENAN SOLUTION (i.e. containing 1.5 grams of carrageenan per 100 millilitres of water).

Fifteen broilers, each one day old, were divided into three groups of five birds each. The first group served as a control group while the chickens of the second group were allowed to expose to Eimeria tenella oocysts suspended at 300 oocysts per millilitre of 1.5% carrageenan solution for about one to two hours daily for two days. The third group was similarly treated except that it was exposed for four days rather than two days. At nine days of age, all birds were challenged with 20,000 oocysts of Eimeria tenella.

### Table 3 - Immunizing chickens with oocysts of Eimeria tenella suspended in a 1.5% carrageenan solution.

| Treatment | First Experiment | | Second Experiment | |
|---|---|---|---|---|
| | Mortality | Average Lesion Score | Mortality | Average Lesion Score |
| Control | 2 | 3.8 | 1 | 3.2 |
| Twice Vaccinated | 0 | 2.7 | 0 | 2.4 |
| Four Times Vaccinated | 0 | 1.9 | 0 | 1.8 |

As is apparent from the results of the experiment set out in Table 3 above, two of the five controlled birds died in the first experiment with a total lesion score averaging about 3.8 per cecum. No mortality was found in the vaccinated groups. The average lesion scores for the twice-vaccinated group was 2.7 per cecum while those vaccinated four times average 1.9 per cecum.

When the experiment was repeated, one bird died in the control group while no birds were lost in the remaining two groups. The average lesion scores were 3.2, 2.4 and 1.8 per cecum for the control, two day exposure and four day exposure respectively.

As shown by Table 1, oocysts of Eimeria tenella can be suspended in a 1.5% carrageenan solution for six months. However, when an experiment was conducted to determine the effect of storage on infectivity of oocysts of Eimeria tenella suspended in a 1.5% carrageenan solution, it was found that infectivity dropped substantially between four and six months (see Example #2).

### EXAMPLE #2

OOCYSTS OF EIMERIA TENELLA WERE SUSPENDED IN A 1.5% CARRAGEENAN SOLUTION, CONTAINING 1.5 GRAMS OF CARRAGEENAN PER 100 MILLILITRES OF WATER, AT A CONCENTRATION OF ABOUT 20,000 OOCYSTS PER MILLILITRE AND STORED AT 4°C.

Six groups of two week old white leghorn cockerels were infected with 0.5 millilitres per bird of the oocysts suspension at 0 hours, 1 month, 2 months, 4 months, 6 months and 8 months after storing.

Freshly collected oocysts caused an average cecal lesion score from both ceca of approximately 7.5. The average lesion scores for the remaining groups were 7.7, 7.8, 8.4, 1.1 and 0.1 for 1 month, 2 months, 4 months, 6 months, and 8 months respectively.

It was found that the 1.5% carrageenan solution worked equally well with other species of chicken coccidia such as Eimeria necatrix (see Example #3).

### EXAMPLE #3

IMMUNIZATION OF CHICKENS AGAINST EIMERIA NECATRIX WITH OOCYSTS SUSPENDED IN 1.5% CARRAGEENAN SOLUTION (i.e. 1.5 GRAMS OF CARRAGEENAN PER 100 MILLILITRES OF WATER).

Fifty day old broiler chickens were divided into two groups of twenty-five birds each. The first group served as a control. Chickens of the second group were allowed to expose to Eimeria necatrix at 100 oocysts per millilitre of 1.5% carrageenan solution for two to three hours on day 6 and again on day 9. Nine days after exposure, fifteen of these twenty-five immunized birds and the same number from the control were challenged with 20,000 oocysts per bird of Eimeria necatrix. The birds were weighed before the challenge and weighed again six days after the challenge. The lesion scores were taken from the jejunum, that part of the intestines where Eimeria necatrix causes the most damage.

Two of the fifteen challenged control birds died but none of the immunized birds died (see Table 4 below). The average lesion scores of the challenged birds were 3.0 and 3.5 for the immunized and control groups respectively.

Table 4 - Immunization of broiler chickens against Eimeria necatrix. Oocysts were suspended at 200 oocysts per millilitre of 1.5% of carrageenan solution (i.e. 1.5 grams of carrageenan per 100 millilitres of water).

| Treatment | Mortality | Average Per Cent Weight Change | Average Lesion Scores |
|---|---|---|---|
| Control, Non-immunized, Challenged | 0/10 | 100% (104 gm) | 0 |
| Immunized, Challenged | 0/10 | 95.2 | 0 |
| Immunized, Challenged | 0/10 | 59.6 | 3.0 |
| Control, Challenged | 2/15 | 55.8 | 3.5 |

Birds were challenged with 20,000 oocysts of Eimeria necatrix 9 days after immunization.

It has been found that, at concentrations of carrageenan below 1.5%, the uniformity of the suspension of the oocysts decreased. While concentrations of carrageenan greater than 1.5% could be used, it would simply be more expensive without increasing the uniformity of suspension of the vaccine. In other words, additional carrageenan would be superfluous.

Large scale tests were carried out on broiler chickens using low levels of oocysts of Eimeria acervulina, Eimeria maxima, Eimeria, necatrix and Eimeria tenella suspended in a 1.5% solution of carrageenan. The results of these tests is set out in Table 5. In all of the tests, the level of Eimeria acervulina, Eimeria maxima, Eimeria necatrix and Eimeria tenella in solution were approximately 1,000, 100, 170 and 170 oocysts per bird respectively at 4 days of age. The carrageenan solution containing the oocysts was poured into water troughs that had been previously dried by cutting off the water supply approximately 1/2 to 1 hour before the suspensions were poured. The birds were exposed again at 7 days of age to Eimeria necatrix and Eimeria tenella, each being at a level of approximately 130 oocysts per bird. The 1.5% carrageenan solution was first made by mixing approximately 57 grams of carrageenan in 3.8 litres of distilled water. The oocysts were added to make approximately 2,000 doses per 3.8 litres. Through tests 1 to 6, one half of the chickens were treated with the vaccine in accordance with the present invention. The remaining half of the birds were medicated with Monensin (a tread mark) in accordance with the manufacturer's instructions. In test #6, vaccination in accordance with the present invention was carried out in an identical manner to tests #1 to #5 except that the vaccine was chilled in a refrigerator overnight prior to use. The temperature of the refrigerator was approximately 4°C.

The results show that there were no significant differences in mortality, average weight gain or in feed conversion between the vaccinated and Monensin medicated birds through tests #1 to #5. However, in test #6, there was a relatively significant difference in the average weight gain. While the vaccine will still work in gel form, this result suggests that the vaccine in the gel form is not as satisfactory as vaccine in the solution form.

Table 5 - Large Scale Tests of Broiler Chickens

| Test No. | Treatment | No. of Chickens Placed | Mortality (%) | (51 days) Average Wt.(lbs) | Feed Conversion |
|---|---|---|---|---|---|
| 1 | Vaccination[a] | 15,000 | 301 (2) | 4.42 | 2.08 |
|   | Monensin (99ppm) | 15,000 | 425(2.8) | 4.48 | 2.09 |
| 2 | Vaccination[a] | 12,000 | 97(0.8) | 4.21 | 2.12 |
|   | Monensin (99ppm) | 12,000 | 87(0.7) | 4.31 | 2.10 |
| 3 | Vaccination[a] | 15,000 | 90(0.6) | 4.37 | 2.11 |
|   | Monensin (99ppm) | 15,000 | 61(0.4) | 4.42 | 2.09 |
| 4 | Vaccination[a] | 15,000 | 588(3.9) | 4.18 | 2.11 |
|   | Monensin (99ppm) | 15,000 | 468(3.1) | 4.22 | 2.11 |
| 5 | Vaccination[a] | 15,000 | 84(0.6) | 4.07 | 2.11 |
|   | Monensin (99ppm) | 15,000 | 221(1.5) | 3.94 | 2.13 |
| 6 | Vaccination[ab] | 15,000 | 468(3.1) | 4.30 | 2.11 |
|   | Monensin (99ppm) | 15,000 | 600(4.0) | 4.58 | 2.08 |

a. The levels of E. acervulina, E. maxima, E. necatrix and E. tenella in the 1.5% carrageenan solution were about 1,000, 100, 170 and 170 oocysts per bird respectively at 4 days of age. The birds were exposed again at 7 days of age to E. necatrix and E. tenella at about 130 oocysts per bird.

b. The mixtures were chilled in the refrigerator (4°C) overnight before use.

Therefore, it is preferred that the vaccine always be used in liquid form as opposed to gel form. All of the birds were kept for 51 days. The mean average weight gain in all of the tests was 4.25 plus or minus 0.02 for the vaccinated birds and 4.27 plus or minus 0.04 for the Monensin medicated birds. The average feed conversion for the vaccinated birds was 2.11 and for the Monensin medicated birds 2.10.

0 243 548

Unlike the battery tests discussed in Examples 1 to 3 of this application, where the birds were raised on wires, tests 1 to 6 of Table 5 were carried out with the birds raised on the floor. Therefore, self-infection or repeated exposure was extremely likely in the latter tests. Because of this probability, lower levels of oocysts were used. For example, in the case of Eimeria tenella, the total number of oocysts used was 300 in the two feedings rather than a multiple of 300.

The level of infectivity of oocysts used in the solution will vary greatly with the type of coccidium or coccidia in the solution. For example, with Eimeria tenella, a level of 5,000 is high because a level of 20,000 is lethal. On the other hand, with Eimeria acervulina, a level of 10,000 is low because a level of 1,000,000 is not lethal. The level of infectivity of the oocysts will also vary with the age and weight of the animals to be treated as well as the exposure time and whether the poultry are raised on the floor or on wire. With Eimeria tenella, a level of infectivity of 100 oocysts per millilitre of solution is preferred for four day old chicks. Other levels can readily be determined by those skilled in the art by reference to the level of a lethal dosage for a particular species of coccidium and by reference to the examples herein.

Since the vaccine can be given readily to the animals to be immunized on more than one occasion. it is not necessary by this method to control the moisture level of the litter or to be concerned with the litter at all. Also, since one or more species of coccidia can be used in the same solution, great flexibility can be achieved while minimizing the change that extraneous species will be introduced into the vaccine.

In field trails using the method and vaccine of the present invention flocks of poultry, exceeding 20,000 birds, were treated simultaneously with little or no mortality, while achieving weight gain and feed conversion at least equal to that of flocks medicated with Monensin.

## Claims

1. A vaccine, which can be taken orally, for immunizing domestic animals of the avian species from coccidiosis characterized by the vaccine comprising oocysts of at least one coccidium in relation to which immunization is desired, said oocysts of said coccidium being diluted in a solution which results in uniform suspension and infection in said oocysts.

2. A vaccine as claimed in Claim 1 characterized in that the solution contains a non-sticky, edible gum.

3. A vaccine as claimed in Claim 2 characterized in that the edible gum solution is approximately a 1.5% solution.

4. A vaccine as claimed in any one of Claims 1, 2 or 3 characterized in that the coccidium is Eimeria tenella.

5. A vaccine as claimed in any one of Claims 2 or 3 characterized in that the edible gum is carrageenan.

6. A vaccine as claimed in any one of Claims 1, 2 or 3 characterized in that the number of species of coccidia ranges from two to six selected from the group consisting of Eimeria necatrix , Eimeria tenella, Eimeria hagani, Eimeria acervulina, Eimeria maxima, Eimeria brunetti , Eimeria proecox, Eimeria mivati and Eimeria mitis.

7. A vaccine as claimed in any one of Claims 2 or 3 characterized in that the edible gum is suspended in water.

8. A vaccine as claimed in any one of Claims 2 or 3 characterized in that the edible gum is suspended in water and the coccidium is Eimeria tenella .

9. A vaccine as claimed in any one of Claims 2 or 3 characterized in that the edible gum is carrageenan and it is suspended in water.

10. A vaccine as claimed in any one of Claims 2 or 3 characterized in that the edible gum is carrageenan, the coccidium is Eimeria tenella and the carrageenan is suspended in water.

11. A vaccine as claimed in any one of Claims 2 or 3 characterized in that the edible gum is carrageenan and the number of species of coccidia ranges from two to six selected from the group consisting of Eimeria necatrix, Eimeria tenella, Eimeria hagani, Eimeria acervulina, Eimeria maxima, Eimeria brunetti, Eimeria proecox, Eimeria mivati and Eimeria mitis.

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 134 703 (UNILEVER N.V.) * Page 1, lines 3-9; claims 1,2,6,7,9,14,28; page 6, lines 8,9,18-20 * | 1-11 | A 61 K 39/012 A 61 K 47/00 |
| X | FR-A-2 408 351 (UNILEVER N.V.) * Claims 1,8,9,12 * | 1,2,7 | |
| A,D | US-A-3 147 186 (S.A. EDGAR) * Claims 1-6 * | 1,4 | |
| A | GB-A-2 059 769 (NISSHIN FLOUR MILLING CO.) | | |

-----

| | |
|---|---|
| | TECHNICAL FIELDS SEARCHED (Int. Cl.4) |
| | A 61 K |

The present search report has been drawn up for all claims

| Place of search | Date of completion of the search | Examiner |
|---|---|---|
| THE HAGUE | 13-11-1986 | CUENDET P.A. |